# EUROPEAN PATENT APPLICATION

(11) **EP 0 865 762 A2**
(43) Date of publication of application: **23.09.1998**
(21) Application number: 98500047.0
(22) Date of filing: 18.02.1998
(51) Int. Cl.: A61B 5/0408

(54) **Electrode support for cardiology**

(30) Priority: 19.02.1997 ES 9700341
(71) Applicant: Pons Creus, Joan Maria, Dr., 08036 Barcelona (ES)
(72) Inventor: Pons Creus, Joan Maria, Dr., 08036 Barcelona (ES)
(74) Representative: Duran Moya, Luis-Alfonso

(57) **Abstract**

The electrode support for cardiology includes an adjustable fastening element on the patient's body which can be fitted and dismantled with ease by means of a quick closure, which fixing element exhibits multiple electrodes which can be stably fixed at variable positions and which exhibit a metal contact zone to establish contact with the patient's body and an attachment for the terminal of the connection conductor with the apparatus intended to perform the electrocardiogram. It permits previous arrangement of the electrodes as well as their rapid fitting and dismantling.

## Description

The present invention relates to an electrode support intended for use in cardiology, which exhibits important characteristics of novelty and inventive activity.

The goal of the electrode support for cardiology as the object of the present invention is to strive to facilitate the means of providing a convenient and rapid device for hospital personnel to enable them to apply the set of electrodes necessary to carry out an electrocardiogram.

The systems presently known for application of electrodes to the patient's body to perform an electrocardiogram include sets of adhesive suckers for fixing the electrodes, which, in many cases, especially in men, requires previous depilation of the zone where they are to be applied, or else single-use adhesive electrodes, which likewise require shaving, all of which leads to heavy cost being incurred and to waste being generated.

The electrode support for cardiology as the object of the present invention is intended to facilitate the means whereby, in a convenient and rapid manner, the electrodes can be applied to the patient's body by means of a fastening element which preferably takes the form of a belt with buckle possibly provided with braces. It is constituted preferably by an element in the form of a strap of several centimetres width produced from an elastic material, which exhibits six reference points distributed over its length and the corresponding mobile electrodes attached to the belt which establish direct skin contact by means of a metal plate and which exhibit an electrical connection on the opposite face.

The application of braces is especially interesting in cases where electrocardiograms (ECG) are carried out in motion.

The electrodes are displaceable along the support, which, as already indicated, will exhibit a number of reference points, for example, in the form of coloured zones to designate the normal positions of the electrodes, which, in most cases, will facilitate rapid and efficient application of the electrodes to perform the electrocardiogram.

The support as the object of the present invention will be likewise applicable to heart disease sufferers requiring electrocardiograms of long duration, such as 24 or 48 hours (Holter). Presently used in these cases are adhesive electrodes with an ample adhesive strap reinforcement for fixing and a cotton mesh adjusted to the patient's thorax, which implies some noticeable inconvenience for the latter. The problems involved in use of the presently known means are likewise accentuated in applied stress tests during which it is necessary to perform electrocardiograms in motion which require a very secure fastening of the electrodes to the patient's thorax, the latter implying a noticeable loss of time for the hospital personnel and a noticeable inconvenience for the patient.

In accordance with the present invention, the electrode support intended for electrocardiograms will essentially incorporate a fastening element integrated by a strap of small width and with elastic characteristics, which will exhibit securely attached braces with clasps to permit their sliding along the latter to achieve adequate positioning in relation to the anatomical characteristics of the patient and exhibiting rapid fitting and dismantling elements, for example, buckles or the like, as well as the waist element and the elements which integrate the braces.

The electrodes will likewise be applied in a sliding manner on the constitutive element of the belt, it being possible to use different versions of the fastening elements.

In a preferred version, the support as the object of the present invention will exhibit a total of six electrodes arranged in one zone with two electrodes and in another zone with four of them, according to the distributions normally used.

In the case where the support is provided with braces, the zone provided with two electrodes will be determined between the anchoring points of the braces in the front part of the fastening element or belt, and the other four electrodes will be situated on one of the sides, specifically that which corresponds to the heart-side zone included between the two anchoring points of the brace which will be on the patient's left.

To fasten the electrodes, it is possible to use any elements whatever which permit sufficiently secure fastening on the device, whether a strap or belt, textile element, etc., likewise allowing direct metal contact of the electrodes with the patient's body and exhibiting a tab in each electrode for their connection to the conductor cable. In a preferred embodiment, the electrode support will be constituted by a metal inlaid unit provided with large openings for passage of the support strap and with a tab intended to receive a demountable contact to perform the electrocardiogram. In this embodiment, the unit will assume a general structure in the form of a tray or noticeably flat contact zone with slightly raised side wings to permit passage of the fastening strap and fixing of the element intended to receive the connection with the apparatus intended to perform the electrocardiogram.

To facilitate better understanding, a number of drawings corresponding to a preferred embodiment of the electrode support for cardiology produced according to the present invention are added by way of an explanatory but non-limiting example.

Fig. 1 shows a perspective view of one electrode support produced according to the present invention showing one of the contact elements.

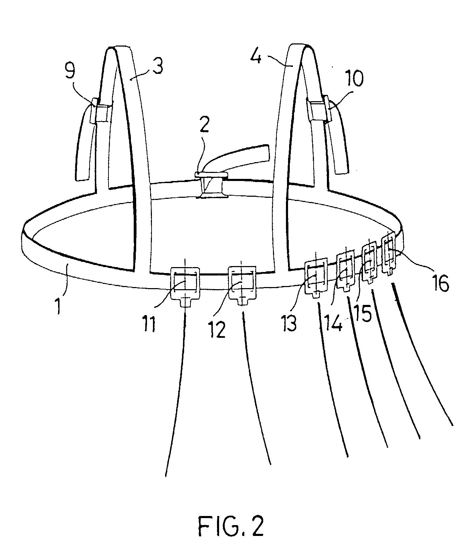

Fig. 2 shows a perspective view in which incorporation of the set of contact elements for the different electrodes is schematically evident.

Fig. 3 shows a front elevation view of an electrode support element.

Figs. 4 and 5 are cross-sectional sketches of the same electrode support element shown in Fig. 3.

Figs. 6 and 7 are typical sketches of application of the electrode support to perform an electrocardiogram, with and without braces respectively.

As is evident from the drawings, the support as the object of the present invention will exhibit an element for fastening to the user's body preferably achieved by means of a relatively narrow elastic strap -1-provided with a quick fixing system, such as a buckle or similar element -2- and possibly exhibiting braces -3- and -4-, the latter being attached at two points on the integrating element -1- of the belt, for example, points -5- and -6- for brace -3- and points -7- and -8- for brace -4-. The said braces will also exhibit individual fixing elements, such as buckles -9- and -10- or similar elements.

The support as a whole will be easy to attach to the user's body, which offers the important advantage of a great deal of the medical personnel's time being saved, moreover permitting a faster application especially recommended in emergencies.

The arrangement of the different electrodes will be achieved on strap -1- in a sliding manner and with means to ensure the stability of arrangement of the same along the said strap or belt -1-.

The arrangement of the electrodes will take place through two of them being arranged in the front part included between the front fastening points of the braces and the other four in a side zone corresponding to the left or heart-side zone of the patient's body.

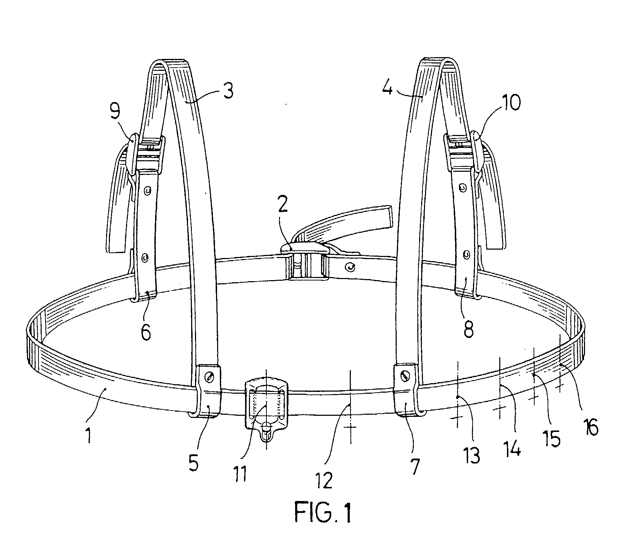

To ensure a faster arrangement of the different electrodes at appropriate locations on the patient's body, belt -1- will carry references to facilitate the said arrangement, being represented by the numbers -11-, -12-, -13-, -14-, -15-, and -16- in Fig. 1.

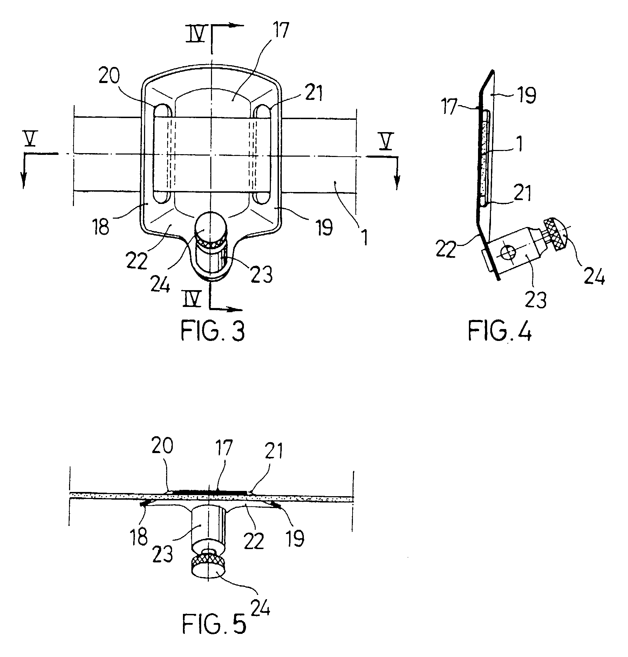

Each of the electrodes will be integrated by a metal unit intended to establish direct contact with the patient's body and which can be easily displaced along support strap -1-, being stable at the points selected for its arrangement. In a preferred embodiment shown in Figs. 3~5, the electrode will exhibit a basically flat part -17-intended to establish contact with the patient's body, which will exhibit easily raised side wings, such as -18-and -19-, with openings -20- and -21- intended to permit passage of support strap -1-. Other easily raised end -22-will permit attachment to a unit -23- for fastening of the connection conductor with the apparatus intended to perform the electrocardiogram, exhibiting, for example, a knurled nut or the like -24- for quick and secure fastening of the terminal tab of the said conductor.

In a concrete embodiment case which has been shown, the electrode support is produced in the form of a die-cast and inlaid tray which enables the different functional zones to be achieved in one unit, i.e. the contact zone with the patient's body, the wings for passage of the support strap, and the fixing zone of the conductor to the apparatus intended to perform the electrocardiogram.

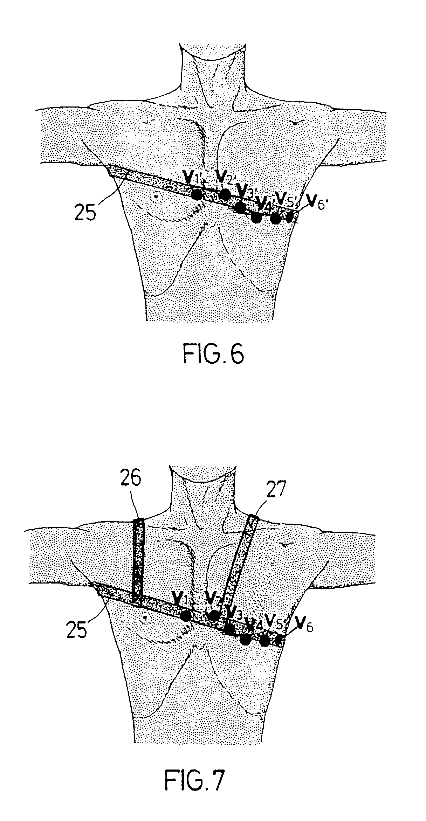

Figs. 6 and 7 show examples of the electrode support being applied to the body of a patient. Fig. 6 shows the support in the form of a single strap -25- in an application preferably intended for use of the device during a relatively reduced period of time and without much mobility of the patient. The different electrodes are indicated by points V₁, V₂, V₃, V₄, V₅, and V₆ representing the most convenient application positions of the electrodes to permit a correct examination.

Fig. 7 shows the arrangement of strap -25-together with braces -26- and -27-, in which case and preferably, the two front electrodes V₁ and V₂ will be arranged between the anchoring points of braces -26- and -27- in the front part.

Said braces -26- and -27- will be arranged to slide along the belt or main strap indicated by number -1-in Fig. 1, being able to include various means to permit sliding and fixing. In a simplified version shown in the attached examples, the braces simply exhibit loops of the same material at their ends with a sliding adjustment, i.e. which can be displaced with minimum force along belt -1- but which are stable once arranged at a desired location.

As will be evident, the support as the object of the present invention will allow multiple design versions of the elements of which it is composed. For example, the fastening elements to the patient's body may have a structure different from that shown, being able to consist, for example, of fabric elements, knitted elements, or the like of variable width but always in such a way that they conform to the essential characteristics of the invention, which include stable application of the support and the possibility of its likewise being stably fixed but with some positional variability of the electrodes per se.

## Claims

1. Electrode support for cardiology, characterised in that it includes an adjustable fastening element on the patient's body which can be fitted and dismantled with ease by means of a quick closure, which fixing element exhibits multiple electrodes which can be stably fixed at variable positions and which exhibit a metal contact zone to establish contact with the patient's body and an attachment for the terminal of the connection conductor with the apparatus intended to perform the electrocardiogram.

2. Electrode support for cardiology according to claim 1, characterised in that the support element exhibits two braces which can be displaced at selected points of the support element to vary its anchorage and which exhibit individual means for their rapid attachment and detachment.

3. Electrode support for cardiology according to claim 1, characterised in that the support element is integrated by a straight elastic strap provided with an element for rapid fixing together of its ends.

4. Electrode support for cardiology according to the previous claims, characterised in that the electrode supports are produced in a metal body exhibiting a flat contact zone with the patient's body, from which extend side wings opposite to each other and opening carriers for passage of the elastic strap comprising the support element and exhibiting a rapid attachment point of the conductor cable for connection with the apparatus intended to perform the electrocardiogram.

5. Electrode support for cardiology according to claim 4, characterised in that the electrode supports are constituted in a die-cast and inlaid metal unit forming a base wall intended to establish contact with the patient's body and raised side wings opposite to each other, two of which exhibit openings for passage of the strap of the fastening element and the other exhibiting an anchoring point for the terminal of the connection conductor with the apparatus intended to perform the electrocardiogram.
